Europäisches Patentamt

European Patent Office

Office européen des brevets

(19)

(11) Veröffentlichungsnummer: **0 207 377**
**A1**

(12) **EUROPÄISCHE PATENTANMELDUNG**

(21) Anmeldenummer: 86108342.6

(22) Anmeldetag: 19.06.86

(51) Int. Cl.4: **G01R 27/26 , G01N 33/44**

(30) Priorität: 24.06.85 DE 3522576

(43) Veröffentlichungstag der Anmeldung:
07.01.87 Patentblatt 87/02

(84) Benannte Vertragsstaaten:
**AT BE DE FR GB IT LU NL SE**

(71) Anmelder: **Siemens Aktiengesellschaft Berlin
und München**
**Wittelsbacherplatz 2**
**D-8000 München 2(DE)**

(72) Erfinder: **Hussain, Amir, Dr. Dipl.-Chem.**
**Bellinzonastrasse 6**
**D-8000 München 71(DE)**
Erfinder: **Pflugbeil, Christa, Dipl.-Ing. (FH)**
**Eichendorff Weg 17**
**D-8021 Hohenschäftlarn(DE)**
Erfinder: **Sinning, Helmut, Ing. grad.**
**Lindenschmittstrasse 3A**
**D-8000 München 70(DE)**

(54) **Messaufbau für dielektrische Spektroskopie.**

(57) Zur Bestimmung elektrischer Kennwerte wie Dielektrizitätszahl, Permeabilitätszahl und Verlustfaktor von Werkstoffen wird ein Meßaufbau vorgeschlagen, der aus einer Temperiereinrichtung (1) mit Regler und Probenaufnahme, einer Meßeinrichtung (4) für die elektrische Impedanz und -daraus abgeleitet -für die Dielektrizitätszahl, Permeabilitätszahl und den Verlustfaktor besteht und des weiteren eine Steuer-Regel-und Recheneinheit (3) sowie eine Ausgabeeinheit (2) aufweist. Mit einem derartigen Meßaufbau ist die Aufnahme und Verarbeitung von Meßdaten als auch die Steuerung der gesamten Meßeinrichtung simultan möglich.

FIG 3

EP 0 207 377 A1

## Meßaufbau für dielektrische Spektroskopie.

Die Erfindung betrifft einen Meßaufbau zur Bestimmung elektrischer Kennwerte wie Dielektrizitätszahl, Permabilitätszahl und Verlustfaktor von Werkstoffen, insbesondere von Kunststoffen. Dieser Meßaufbau soll im folgenden als dielektrisches Spektrometer bezeichnet werden.

In der Anwendungstechnik ist es erforderlich, die Eigenschaften der verwendeten Werkstoffe in Abhängigkeit von der Temperatur, Zeit und Frequenz zu charakterisieren. Dabei interessieren vor allem die Dielektrizitätszahl, die Permeabilitätszahl und der Verlustfaktor in Abhängigkeit von der Frequenz, der Temperatur und der Zeit.

Durch diese Werte wird nämlich die Eignung von Werkstoffen für den jeweiligen Einsatz bestimmt. Das ermittelte Eigenschaftsprofil dient demnach der Charakterisierung von Werkstoffen für diverse Anwendungen, insbesondere für die Elektronik. Außerdem ist es von wesentlicher Bedeutung für die Eingangs-, Verarbeitungs-und Qualitätskontrolle eines Produkts.

Bisher wurden die elektrischen Eigenschaftswerte manuell erfaßt und berechnet. Darüber hinaus wird in der Regel nur bei Raumtemperatur und in Abhängigkeit von der Frequenz zum Beispiel die Dielektrizitätszahl und der Verlustfaktor bestimmt. Mit einem Meßpunkt kann aber kein Aufschluß über das Werkstoffverhalten bei anderen Temperaturbedingungen gefunden werden.

Aus Research Report R 80 -2 MIT vom März 1980 ist es bekannt, daß man obige Werte bei verschiedenen Temperaturen ermitteln kann, um Anhaltspunkte für die Beurteilung eines Werkstoffes zu erhalten. Das dort angewendete Verfahren hat aber große Nachteile in bezug auf die Meßergebnisse und den Werkstoff selbst.

Wird die Messung im instationären Zustand ausgeführt, ist die Interpretation der gefundenen Werte wegen des Zeitverzugs bei thermischen Ausgleichsvorgängen mit großer Unsicherheit behaftet.

Wird andererseits die zum Temperaturausgleich erforderliche Zeit abgewartet, ist eine Schädigung der Probe durch zu lange Temperatureinwirkung nicht auszuschließen.

Eine weitere Unsicherheit ergibt sich aus der Tatsache, daß meistens die Prüftemperatur nicht an der Probe selbst erfaßt wird.

Der Erfindung liegt die Aufgabe zugrunde, einen Meßaufbau zu konzipieren, durch den in kleinen Zeiteinheiten zu vorgegebenen Parametern die in der Einleitung genannten Kennwerte ermittelt werden. Unter kleinen Zeiteinheiten sind beispielsweise < 2 sec zu verstehen. Das zu schaffende dielektrische Spektrometer ist für die Eingangs-, Verarbeitungs-und Qualitätskontrolle in der Forschung, Entwicklung und Fertigung von großer Bedeutung.

Die der Erfindung zugrundeliegende Aufgabe wird dadurch gelöst, daß für einen Aufbau gemäß dem Oberbegriff eine thermische Einrichtung mit Regler und mit Probenaufnahme, eine Meßeinrichtung für die elektrische Impedanz und -daraus abgeleitet -für die Dielektrizitätszahl, die Permeabilitätszahl und den Verlustfaktor eine Steuer-, Regel-und Recheneinheit und eine Ausgabeeinheit, zum Beispiel Plotter, vorgesehen sind. Mit einem derartigen dielektrischen Spektrometer ist sowohl die Aufnahme und Verarbeitung von Meßdaten als auch die Steuerung der gesamten Meßeinrichtung simultan möglich. Manuell wäre eine derart große Meßdatenrate pro Zeiteinheit und daraus resultierende hohe Auflösung nicht zu bewerkstelligen. Außerdem können mit einem entsprechenden Meßaufbau das Alterungsverhalten und Vernetzungsreaktionen von Werkstoffen untersucht und damit die Verarbeitungsparameter ermittelt und optimiert werden.

Mit Hilfe des erfindungsgemäßen dielektrischen Spektrometers ist es möglich, innerhalb kleiner Zeitintervalle vorgenannte elektrische Kennwerte in Abhängigkeit von Temperatur, Zeit und Frequenz zu ermitteln. Hierdurch wird eine eindeutige Zuordnung der erfaßten Meßwerte zu den gewählten Parametern (wie zum Beispiel Temperatur und/oder Frequenz) erreicht. Es ist von großer Bedeutung, die Meßwerte sekundenschnell zu erfassen, um die erforderliche hohe zeitliche Auflösung zu erzielen. Voraussetzung hierfür ist eine optimale Abstimmung von Temperiereinrichtung, Probenaufnahme und Meßeinrichtung sowie von Steuer-, Regel-, Rechen-und Ausgabeeinheit aufeinander.

Bei der großen Bedeutung, die der Temperatur in bezug auf die elektrischen Eigenschaftswerte eines Werkstoffes zukommt, ist es unerläßlich, die Temperaturabhängigkeit dieser Werte zu ermitteln. Dadurch kann der thermische Einsatzbereich abgesteckt werden. Wichtig für die Zuordnung der gemessenen elektrischen Werte zur entsprechenden Temperatur ist eine genaue und schnelle Erfassung der Probentemperatur. Dies ist nur möglich, wenn die Probenaufnahme zugleich zur Aufnahme des Temperaturfühlers eingerichtet ist. Um die Ist-Temperatur, das heißt die Probentemperatur, möglichst nahe an der Solltemperatur zu führen, wird dieser Temperaturfühler auch zur Regelung der Temperiereinrichtung verwendet. Dadurch wird die Temperaturausgleichszeit möglichst klein gehalten und eine thermische Schädigung des Probenmaterials verhindert. Ohne diese aufeinander abgestimmte Anordnung wäre es nicht möglich, gute Auflösung und reproduzierbare Werte zu erhalten.

Wie aus dem noch dargestellten Ausführungsbeispiel hervorgeht, sind geringe Verarbeitungsunterschiede bezüglich ihrer Auswirkung auf das elektrische Verhalten eines Werkstoffs mit Hilfe des dielektrischen Spektrometers erfaßbar.

Zusätzlich zur Temperaturabhängigkeit ist die Kenntnis der Frequenzabhängigkeit der elektrischen Kennwerte für die Beurteilung eines Werkstoffes von Bedeutung.

Mittels unterschiedlicher Probenabmessungen, entsprechend ausgebildeter Kontaktierung innerhalb der Probenaufnahme und einer geeigneten Meßeinrichtung ist es mit einem Meßaufbau nach der Erfindung möglich, einen Frequenzbereich von 0 Hz bis 1 GHz zu überstreichen.

Wie im Ausführungsbeispiel dargestellt, läßt sich nur bei gleichzeitiger Berücksichtigung von Temperatur und Frequenz eine eindeutige Materialbewertung vornehmen.

Das Verfahren nach der Erfindung wird noch an einem Ausführungsbeispiel in einzelnen Schritten erläutert.

Des weiteren soll für optimale Werkstoffauswahl das jeweilige Alterungsverhalten bekannt sein. Mit Hilfe des angepaßten Meßaufbaus können Alterungsbedingungen simuliert und über die gemessenen elektrischen Werte auf das Alterungsverhalten geschlossen werden.

Von großer Bedeutung sowohl für die Entwicklung als auch in der Fertigung ist die Kenntnis von Vernetzungsreaktio nen und deren zeitlicher Verlauf bei festgelegten Bedingungen.

Mit Hilfe des dielektrischen Spektrometers gelingt es, diese Reaktionen zeitlich anhand der Veränderungen der gemessenen elektrischen Werte zu verfolgen. Dadurch können Verarbeitungsparameter entsprechend angepaßt und somit die Vernetzung optimiert werden.

In ähnlicher Weise kann das dielektrische Spektrometer auch in der Prozeßkontrolle eingesetzt werden. In situ, das heißt simultan mit dem Verfahrensablauf, wird aufgrund von Veränderungen der elektrischen Eigenschaften des zu verarbeitenden Werkstoffs der Produktionsablauf gesteuert.

Die Erfindung wird anhand der Figuren und eines Ausführungsbeispiels erläutert. Es zeigen:

Figur 1 den Kurvenverlauf Dielektrizitätszahl $\epsilon_\gamma$ und Verlustfaktor D von mehreren Proben unterschiedlicher Behandlung in Abhängigkeit von der Temperatur bei einer Frequenz,

Figur 2 den Kurvenverlauf $\epsilon_\gamma$ und D von einer Probe in Abhängigkeit von der Temperatur bei mehreren Frequenzen und

Figur 3 ein Funktionsschema des dielektrischen Spektrometers.

Ausführungsbeispiel:

Als Proben wurden beidseitig mit Kupfer-Folie kaschierte Kunststoffolien verwendet. Die Kupferfolie dient als Kontaktelektrode. Dadurch wird jeglicher Luftspalt, der das Ergebnis verfälschen würde, zwischen eigentlicher Probe und Kontaktierung verhindert. Die Proben waren vor der Messung unterschiedlichen Temperatur-Zeit-Bedingungen ausgesetzt.

Nach dem elektrischen Abgleich der Meßeinrichtung wurde der Prüfling mittels der Probenaufnahme elektrisch kontaktiert und in die Temperierkammer, die zum Beispiel Heizen bis 350°C und Kühlen bis minus 150°C kann, eingegracht. Die Temperiereinrichtung ist so aufgebaut, daß der Innenraum während der Messung mit Inertgas gespült werden kann. Im Bedarfsfall kann die Messung auch im Vakuum erfolgen. Vorher wurden noch die Abmessungen (Fläche, Dicke) des Probekörpers ermittelt. Zur Temperaturbestimmung und -regelung wurde der Fühler in die Probenaufnahme eingeführt. Über die zentrale Rechen-, Steuer -und Regeleinheit werden die gewüschte Meßart (im Beispiel Bestimmung von Kapazität und Verlustfaktor), das Temperaturprofil und die Meßfrequenzen im Dialogverfahren vom Benutzer vorgegeben.

Mit Hilfe des angepaßten Reglers wird der gewünschte Temperaturzyklus durchfahren und mittels der Meßeinrichtung werden die erforderlichen Messungen in kleinen Zeitabständen ausgeführt. Zu jedem elektrischen Meßpunkt wird somit die zugehörige Temperatur erfaßt. Alle so erhaltenen Daten werden nach entsprechender Aufbereitung an der Ausgabeeinheit dargestellt.

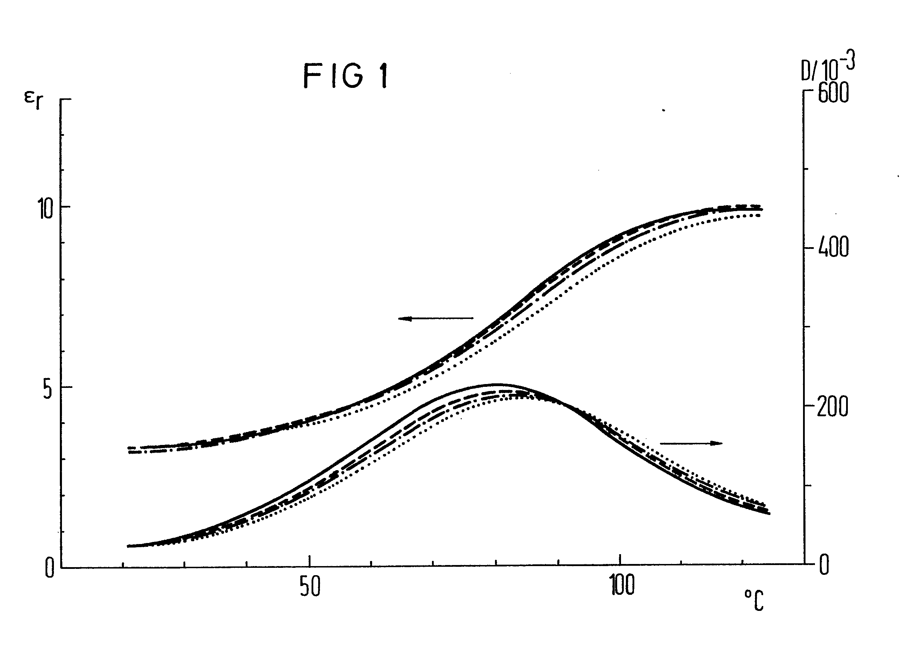

In der Figur 1 ist der Kurvenverlauf für eine Probe, die eine halbe Stunde bei 200°C vorbehandelt wurde, mit einer ausgezogenen Linie gekennzeichnet. Mit gestrichelter Linie ist eine Probe wiedergegeben, die eine Stunde bei 200°C vorbehandelt wurde. Eine strichpunktierte Linie zeigt den Kurvenverlauf für eine Probe, die eineinhalb Stunden bei 200°C vorbehandelt wurde. Ein punktierter Kurvenverlauf gilt für eine Probe, die zwei Stunden bei 200°C vorbehandelt worden ist.

Die oberen Kurven sind der Dielektrizitätszahl $\epsilon_\gamma$ und die unteren Kurven dem Verlustfaktor D zugeordnet. Die Pfeile in der Figur geben einen entsprechenden Hinweis.

Aus der Figur 1 ist zu ersehen, daß geringe unterschiedliche Vorbehandlungen des Probenmaterials bereits erhebliche Abweichungen in den elektrischen Kennwerten bewirken.

Diese kleinen Materialunterschiede sind mit anderen Meßmethoden bisher nicht mit dieser Genauigkeit und Reproduzierbarkeit erfaßbar gewesen.

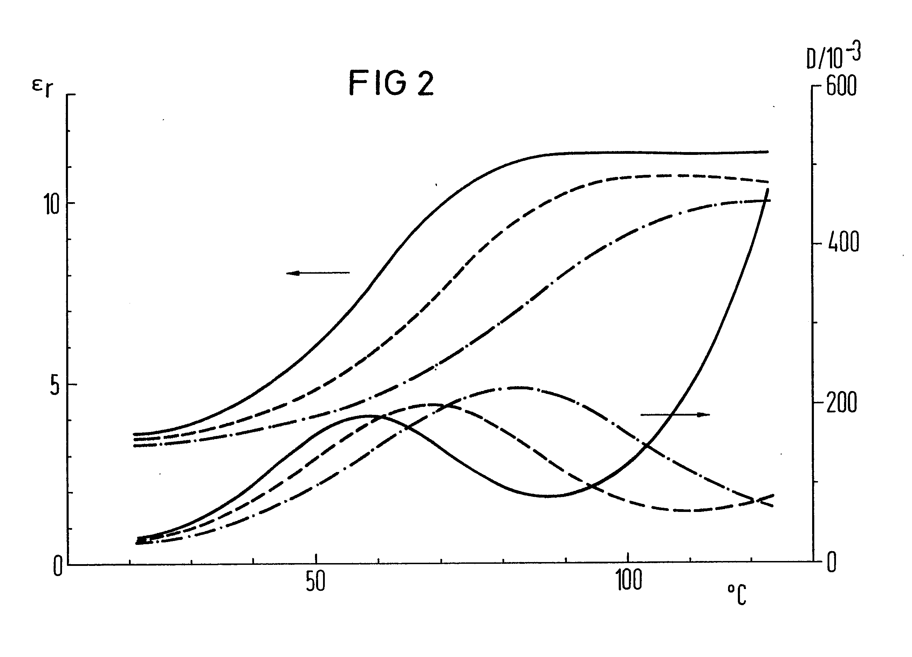

In der Figur 2 ist die starke Abhängigkeit der Dielektrizitätszahl und des Verlustfaktors der Probe von der Frequenz über der Temperatur dargestellt. In dieser Figur sind die Kurven der Probe, die bei 200°C eine Stunde vorbehandelt wurde, bei den Frequenzen 1, 10 und 100 kHz dargestellt. Die durchgezogene Linie entspricht dem Kurvenverlauf bei 1 kHz, die gestrichelte Linie dem Verlauf bei 10 kHz und die strichpunktierte Linie dem Kurvenverlauf bei 100 kHz. Die oberen Kurven sind der Dielektrizitätszahl zugeordnet und die unteren dem Verlustfaktor.

Die aus beiden Bildern gewonnenen Daten führen zu einer Optimierung der Vorbehandlungs- bzw. der Verarbeitungsbedingungen und damit zu einem verbesserten und gleichbleibenden Endprodukt.

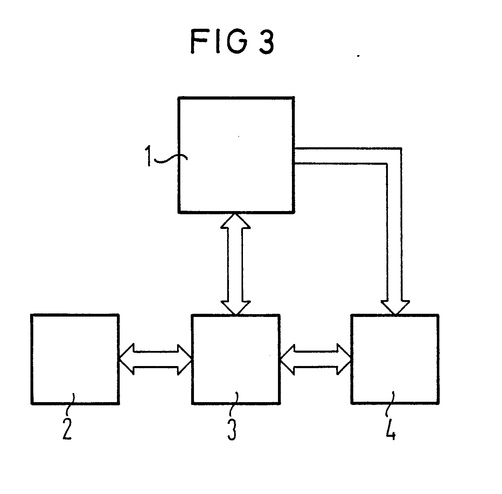

In Figur 3 ist das Funktionsschema des dielektrischen Spektrometers wiedergegeben. Mit 1 ist die Temperiereinrichtung nebst Probenaufnahme bezeichnet; 2 kennzeichnet den Plotter, 3 die Datenverarbeitungsanlage und 4 die Meßeinrichtung.

Die Probenaufnahme besteht aus einem Plattenkondensator für feste Stoffe, der für Flüssigkeiten und Pasten aber topfförmig ausgebildet ist. An dem Plattenkondensator ist direkt der Temperaturfühler für die Regelung der Temperiereinrichtung angebracht. Die Meßeinrichtung besteht aus einer Kapazitätsmeßbrücke. Diese Meßbrücke gilt vorzugsweise bis zu einer Frequenz von 10 MHz. Darüber hinaus findet eine Impedanzmeßeinrichtung Verwendung mit einer Probenhalterung in Form einer koaxialen Meßzelle.

Bei der Verwendung der Kapazitätsmeßbrücke wird die elektrische Verbindung zwischen der Probenhalterung und der Meßbrücke mit Hilfe einer Vierdrahtmeßmethode durchgeführt. Nur dadurch ist die Reproduzierbarkeit der Meßwerte in engeren Toleranzgrenzen gewährleistet.

## Ansprüche

1. Meßaufbau zur automatischen Bestimmung elektrischer Kennwerte von Werkstoffen, insbesondere von Kunststoffen, **dadurch gekennzeichnet**, daß zur Bestimmung der Dielektrizitätszahl, der Permeabilitätszahl und des Verlustfaktors in kleinen Zeiteinheiten eine Temperiereinrichtung (1) mit Regler und mit Probenaufnahme, daran anschließend eine Meßeinrichtung (4) für Dielektrizitätszahl, Permeabilitätszahl und Verlustfaktor, ferner eine Steuer-, Regel-und Recheneinheit (3) verbunden mit einer Ausgabeeinheit (2), zum Beispiel Plotter, vorgesehen sind.

2. Meßaufbau nach Anspruch 1, **dadurch gekennzeichnet**, daß die Probenaufnahme auch zur Aufnahme eines Temperaturfühlers dient.

3. Meßaufbau nach Anspruch 1, **dadurch gekennzeichnet**, daß die Meßeinrichtung zur Erfassung der Kenndaten in niederfrequentem und hochfrequentem Bereich ausgebildet ist.

4. Verfahren zur automatischen Bestimmung elektrischer Kennwerte mit einem Meßaufbau nach Anspruch 1, **dadurch gekennzeichnet**, daß nach dem Abgleich der Meßeinrichtung, Kontaktieren der Probe und Einbringen in die Temperiereinrichtung, in die Steuer-, Regel-und Recheneinheit das gewünschte Temperaturprofil (umfassend die Heiz-, Kühlraten und die Haltezeiten), die Meßart, die Meßfrequenzen und die Starttemperatur eingegeben werden, daß beim Durchfahren des Temperaturprofils laufend die geforderten elektrischen Messungen durchgeführt, simultan dazu die Probentemperatur erfaßt und die Ergebnisse an die Ausgabeeinheit ausgegeben werden.

5. Verfahren nach Anspruch 4, **dadurch gekennzeichnet**, daß die Probentemperatur zur Steuerung des Verfahrensablaufs dient.

6. Verfahren nach Anspruch 4 zur Bestimmung des Alterungsverhaltens von Werkstoffen, **dadurch gekennzeichnet**, daß nach dem Abgleich der Meßeinrichtung Kontaktieren der Probe und Einbringen in die Temperiereinrichtung, in die Steuer-, Regel-und Recheneinheit die Alterungsbedingungen, die Temperatur, die Dauer, die Meßart und die Meßfrequenzen eingegeben werden und daß nach bestimmten Zeiteinheiten die geforderten elektrischen Messungen durchgeführt und die Ergebnisse in Abhängigkeit vorgewählter Umgebungsbedingungen an die Ausgabeeinheit ausgegeben werden.

7. Verfahren nach Anspruch 4 zur Bestimmung der Vernetzungsreaktionen von Werkstoffen, **dadurch gekennzeichnet**, daß nach dem Abgleich der Meßeinrichtung, Kontaktieren der Probe und Einbringen in die Temperiereinrichtung, in die Steuer-, Regel-und Recheneinheit die Vernetzungsbedingungen, die Temperatur, die Dauer, die Meßart und die Frequenzen eingegeben werden und daß nach bestimmten Zeiteinheiten die geforderten elektrischen Messungen durchgeführt und die Ergebnisse in Abhängigkeit eventueller Parameter an die Ausgabeeinheit ausgegeben werden.

8. Verfahren nach den Ansprüchen 4 bis 6, **dadurch gekennzeichnet**, daß in Abhängigkeit von den ausgegebenen Meßwerten Verarbeitungsparameter und Produktionsablauf gesteuert und überwacht werden.

FIG 1

FIG 2

# FIG 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | REVUE DE PHYSIQUE APPLIQUEE, Band 15, Nr. 10, Oktober 1980, Seiten 1563-1570, Paris, FR; A. MOLITON et al.: "Spectromètre de mesures diélectriques automatisé dans le domaine de température (77 K-380 K) et dans l'intervalle continu de fréquence (500 Hz-1 MHz)" * Seite 1564, Abschnitt 2; Seite 1567, Abschnitt 5; Abbildungen 1,5 * | 1-5 | G 01 R 27/26 G 01 N 33/44 |
| X | US-A-4 399 100 (A. ZSOLNAY) * Spalte 3, Zeilen 35-42,57-64; Abbildung 1 * | 1 | |
| A | US-A-4 064 455 (E.L. HOPKINS) * Spalte 2, Zeilen 34-46; Spalte 2, Zeile 60 - Spalte 3, Zeile 2 * | 4-7 | RECHERCHIERTE SACHGEBIETE (Int. Cl.4) |
| A | DE-A-2 949 381 (A/S N. FOSS ELECTRIC) * Seiten 6-8; Abbildung 1 * | 1 | G 01 R 27/00 G 01 N 33/00 G 01 N 27/00 G 01 N 17/00 |

Der vorliegende Recherchenbericht wurde für alle Patentansprüche erstellt.

| Recherchenort | Abschlußdatum der Recherche | Prüfer |
|---|---|---|
| DEN HAAG | 29-09-1986 | DELPORTE B.P.M. |